(19) Europäisches Patentamt
European Patent Office
Office européen des brevets

(11) **EP 3 332 819 A1**

(12) **EUROPEAN PATENT APPLICATION**
published in accordance with Art. 153(4) EPC

(43) Date of publication:
**13.06.2018 Bulletin 2018/24**

(51) Int Cl.:
**A61M 5/145** (2006.01)

(21) Application number: **16832262.6**

(22) Date of filing: **28.07.2016**

(86) International application number:
**PCT/CN2016/092122**

(87) International publication number:
**WO 2017/020774 (09.02.2017 Gazette 2017/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **03.08.2015 CN 201510464604**

(71) Applicant: **Chongqing Beka Med, Inc.**
**Xiema Town**
**Beibei District**
**Chongqing 400712 (CN)**

(72) Inventor: **HE, Xiaojun**
**Geleshan town**
**Shapingba district**
**Chongqing 400036 (CN)**

(74) Representative: **Müller-Boré & Partner**
**Patentanwälte PartG mbB**
**Friedenheimer Brücke 21**
**80639 München (DE)**

(54) **SYRINGE**

(57)    The disclosure discloses an injection device. The injection device comprises: a drug storer having a first space for storing a drug; a unidirectional switch having a second space in fluid communication with the first space; and an injection body having a third space in fluid communication with the second space, wherein the third space is provided with a piston adapted to move along the injection body, and to pump the drug stored in the first space into the third space. The drug storer comprises a base and a head cover, and at least one of the base and the head cover is formed by a flexible film, thereby forming the first space (101) for storing a drug.

Fig. 5

**Description**

**TECHNICAL FIELD**

The disclosure relates to an injection device, and in particular to an injection device having a film liquid drug storage cavity.

**BACKGROUND**

At present, the volume utilization rate for a liquid drug in a cylindrical syringe drug storage cavity is low due to its shape characteristic. In the case of a given dosage, a designed drug pump generally has a large size. Prolonged use of a large drug pump will bring inconvenience to a patient' s life, so that an oval syringe drug storage cavity attracts attention. However, although the oval syringe has improved the device size, the improvement is very limited. Moreover, due to stress changes along the circumference of the oval syringe, drug leakage occurs frequently. The requirements for machining precision of parts are also higher, thereby increasing the design difficulty and cost. Therefore, an improved liquid drug injection device is in urgent need.

**SUMMARY**

In order to solve the technical problem, the disclosure provides an improved injection device.

According to an embodiment of the disclosure, the injection device includes: a drug storer having a first space for storing a drug; a unidirectional switch having a second space in fluid communication with the first space; and an injection body having a third space in fluid communication with the second space, wherein the third space is provided with a piston adapted to move along the injection body, and to pump the drug stored in the first space into the third space.

According to the aspect of an embodiment, the drug storer includes a base and a head cover, wherein at least one of the base and the head cover is formed by a flexible film, thereby forming the first space.

According to the aspect of an embodiment, the base has a concave, and is a hard plastic base formed by polypropylene. The head cover is formed by a flexible film, which is pasted to the base by a hot pressing technology, thereby enabling the film and the concave to form the first space for storing a drug.

According to the aspect of an embodiment, both the base and the head cover are formed by the flexible film, and are pasted by a hot pressing technology to form the first space for storing a drug.

According to the aspect of an embodiment, the second space has a one-way valve arranged on a side proximate to the storage cavity in the second space.

According to the aspect of an embodiment, on a side of the second space distal to the storage cavity, a constant force providing device connected to the piston is arranged to push the piston to discharge the drug pumped into the third space.

According to the aspect of an embodiment, the constant force providing device includes a constant force spring, and the second space has a first operation state and a second operation state. In the first operation state, a pressure difference between the first space and the injection body is formed by pulling the constant force spring to turn on the unidirectional switch and pump the drug stored in the first space into the third space of the injection body; and in the second operation state, the compressed constant force spring provides a constant urging force to the piston, causing the piston to move towards a direction of the second space, and increasing the pressure in the second space. Due to the pressure difference between the second space and the first space, the unidirectional switch is turned off under a pressure in a forward direction. Moreover, the piston moves to push out a drug pumped thereinto.

According to the aspect of an embodiment, the unidirectional switch includes a sealing gasket arranged along an extension direction of the second space, a steel ball and a spring. The sealing gasket has an opening; and the steel ball is arranged against the spring, and seals the opening under the action of the spring.

According to the aspect of an embodiment, the constant force spring includes a sleeve, and a first spring and a second spring arranged inside the sleeve. The first spring has a smaller cross-section than a cross-section of the second spring, so that when the first spring and the second spring are compressed, the compressed first spring is compressed within the compressed second spring. The first spring and the second spring have an identical stiffness coefficient.

According to the aspect of an embodiment, the constant force spring has a small stiffness coefficient and a high compression ratio to ensure the spring having a very short operating stroke in the working process, thereby achieving the purpose of an approximate constant force.

According to an embodiment of the disclosure, the injection device further includes a flow rate limiter arranged in a drug fluid receiving direction of an injection body for limiting a flow rate of a liquid drug pushed out from the injection body.

According to the aspect of an embodiment, the flow rate limiter has a capillary structure to precisely and constantly effect the flow rate of the liquid drug by utilizing a flow rate limiting effect of the capillary.

According to the aspect of an embodiment, the flow rate limiter includes: a tubule for receiving the liquid drug pushed out from the injection body; and a column arranged in the tubule to form a gap with the inner wall of the tubule allowing the received liquid drug to pass through. The length of the column within the tubule is preset, thus adjusting the flow rate of the liquid drug passing through the gap.

According to an embodiment of the disclosure, the injection device further includes a quantifying syringe arranged in a drug fluid receiving direction of the injection body for accommodating and controlling a total quantity of a liquid drug pushed out from the injection body.

According to the aspect of an embodiment, the quantifying syringe includes: a volume body provided with a first passage and a second passage on both sides thereof; a fluid pipe having a fluid passage in fluid communication with the volume body through the first passage and the second passage; a pull rod provided with a center hole, an opening in fluid communication with the center hole, and sealing rings; a pressurized liquid inlet in fluid communication with the fluid passage or the center hole; and a normal pressure liquid outlet; wherein the pull rod moves along a longitudinal direction of the fluid passage, a gap is formed between the pull rod and the fluid passage, and a drug accommodation cavity is formed by the sealing rings between the pull rod and the fluid pipe. The normal pressure liquid outlet is in fluid communication with the fluid passage, so that a liquid drug discharged from the volume body to the cavity via the first passage and the second passage is discharged through the normal pressure liquid outlet.

According to the aspect of an embodiment, the volume body includes a first casing having a concave, a second casing having a concave, and a film arranged between the first casing and the second casing. The film isolates the volume body into a first volume body and a second volume body.

According to the aspect of an embodiment, when the pull rod is pushed, a pressurized liquid flows into the second volume body through the opening to push the film to move towards the first volume body, thus discharging a liquid accommodated in the first volume body through the first passage, and discharging the liquid discharged from the first volume body through the liquid outlet via the cavity. When the pull rod is pulled and then released, a pressurized liquid flows into the first volume body through the fluid passage to push the film to move towards the second volume body, thus discharging a liquid accommodated in the second volume body through the second passage, and discharging the liquid discharged from the second volume body through the liquid outlet via the cavity.

According to an embodiment of the disclosure, the injection device may further include a drug accommodation cavity in fluid communication with the injection body through a control valve, wherein a liquid drug in the drug accommodation cavity is controlled by the control valve and controllably filled in the injection body.

According to the aspect of an embodiment, the control valve may include a cavity and a second piston arranged in the cavity. A plurality of sealing rings may be arranged between the second piston and the cavity, and a spring device may be arranged at an end of the second piston, wherein when the second piston is pressed at a second end of the second piston, the spring device may be compressed, and the drug accommodation cavity is in fluid communication with the injection body through a gap between the sealing rings; and when the piston is released, the compressed spring device drives the second piston to return to an initial position, thus isolating the drug accommodation cavity from the injection body through the second sealing ring.

According to the aspect of an embodiment, the injection device may further include an indwelling needle catheter in fluid communication with the injection body through the control valve. When the second piston is pressed at a second end of the second piston, the spring device is compressed, the drug accommodation cavity is in fluid communication with the injection body through the gap between the second sealing ring and the third sealing ring, and the indwelling needle catheter is respectively isolated from the drug accommodation cavity and the injection body through the third sealing ring; and when the second piston is released, the compressed spring device drives the second piston to return to the initial position, thus isolating the drug accommodation cavity from the injection body through the second sealing ring, and the indwelling needle catheter is in fluid communication with the injection body through the gap between the second sealing ring and the third sealing ring.

According to the aspect of an embodiment, an injection system is provided. The injection system distributes the drug in the drug storer through the unidirectional switch to the injection body to realize large volume of quantitative output through a small size.

## BRIEF DESCRIPTION OF THE DRAWINGS

Fig. 1 is a schematic diagram showing an injection device according to an embodiment of the disclosure;

Fig. 2 is a partial enlarged drawing showing a drug storer according to an embodiment of the disclosure;

Fig. 3 is a partial enlarged drawing showing a drug storer according to another embodiment of the disclosure;

Fig. 4 is an enlarged schematic diagram of a one-way valve according to an embodiment of the disclosure;

Fig. 5 is a schematic diagram showing an injection device having a quantifying syringe and a flow rate limiter according to an embodiment of the disclosure;

Fig. 6 is a schematic diagram showing a constant force providing device according to an embodiment of the disclosure;

Fig. 7 is a schematic diagram showing a spring for a constant force providing device according to an embodiment of the disclosure;

Fig. 8 is an enlarged schematic diagram showing a flow rate limiter according to an embodiment of the disclosure;

Fig. 9 is a state diagram showing a quantifying syringe when pushing a spring pull rod according to an embodiment of the disclosure;

Fig. 10 is a state diagram showing a quantifying syringe when pulling a spring pull rod according to an embodiment of the disclosure;

Fig. 11 shows a schematic diagram of a refillable spring-driven pump controlled by a control valve; and

Fig. 12 shows another embodiment of the spring-driven pump according to Fig. 11.

## DETAILED DESCRIPTION OF EMBODIMENTS

The disclosure is further described in detail below in conjunction with the accompanying drawings and the embodiments. The embodiments described herein are only used for illustrating the invention, other than limiting the invention. Moreover, it is further necessary to explain that for the purpose of description, the accompanying drawings only show relevant parts related to the invention. Furthermore, in the scope of inspiration disclosed in the disclosure, features in the embodiments of the disclosure may be mutually combined, unless a conflict between technical solutions will be caused during mutual conflict. The disclosure is described in detail below by referring to the accompanying drawings and in conjunction with the embodiments.

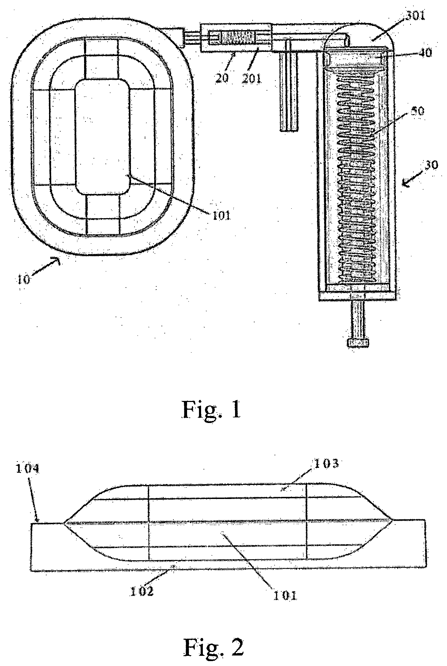

Fig. 1 is a schematic diagram showing an injection device according to an embodiment of the disclosure. As shown in Fig. 1, the injection device may include:

a drug storer 10 having a first space 101 for storing a drug;

a unidirectional switch 20 having a second space 201 in fluid communication with the first space 101 and being provided with a one-way valve; and

an injection body 30 having a third space 301 in fluid communication with the second space 201 and being provided with a piston 40 adapted to move along the injection body 30,

wherein the unidirectional switch 20 is arranged on a side proximate to the drug storer 10 in the second space 201, and a constant force providing device 50, such as a constant force spring, is arranged on an opposite side distal to the drug storer 10 in the second space 201.

Fig. 2 is a partial enlarged drawing showing a drug storer 10 according to an embodiment of the disclosure.

The drug storer 10 includes a base 102 and a head cover 103. The base 102 may be a hard plastic base formed by, e.g. , polypropylene, and has a concave structure for forming a first space 101 accommodating a liquid drug therein. The head cover 103 may be a soft plastic film. A sealing paste is formed between the base 102 and the head cover 103 by a hot pressing method. A film drug storage cavity (i.e. , the first space 101) has a liquid drug inlet and a liquid drug outlet, and is in fluid communication with the second space 201 of the unidirectional switch 20 through the inlet and the outlet.

The drug storage cavity 101 thus formed may greatly increase the effective volume utilization rate to reduce the volume of the device at a given drug volume. Moreover, because the top, i.e., the head cover 103, of the container is a soft plastic film, and is very easily deformed, the resistance is very small when a drug is extracted from the container cavity. This is very beneficial to designing a refillable member and operating a one-way valve.

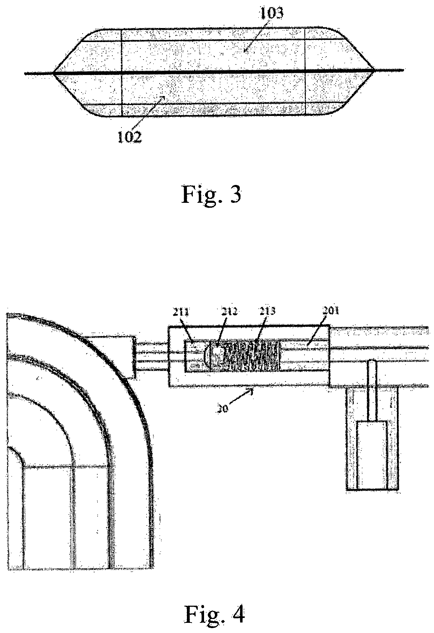

Fig. 3 shows a partial enlarged drawing of a drug storer according to another embodiment of the disclosure. In the embodiment shown in Fig. 3, both the base 102 and the head cover 103 are made of a film, thereby forming a drug storer 10 having a double-faced film structure. The drug storer 10 having a double-faced film structure can realize seamless fitting, thereby obtaining a more efficient drug utilization rate.

In an embodiment, the film drug storer 10 may store doses of, e.g., 3 to 6 days, while a syringe drug injection body only stores doses of, e.g., 1 day. A patient only needs to recharge the syringe drug injection body (recharge a liquid drug and potential energy of a spring) by pulling a piston (e.g. , pulling a pull rod arranged in the piston) once every day. The whole device may provide a patient with a drug demand of, e.g., 4 days to 1 week.

Furthermore, the drug storage cavity fundamentally changing the volume utilization rate may be a non-barrel-shaped. The film drug storer formed by a hot pressing technology improves the volume rate to the greatest extent. The soft film is pasted by hot pressing to a casing formed by hard plastic injection molding, which may obtain a few advantages as follows:

the volume rate is greatly improved;

the volume and shape may be freely changed based on design requirements;

the film deformation resistance is almost zero, and the pressure in the container may be considered as constant (barometric pressure);

the manufacturing process is mature and reliable;

the airtightness and safety may be simply tested and verified; and

both injection molding and hot pressing are low-cost technologies that may achieve quantity production.

The third space 301 of the injection body 30 has a first operation state and a second operation state. In the first operation state, a piston 40, pulled by an external force, compresses a constant force providing device 50 to move towards a side of the injection body 30 proximate to the piston pull rod, causing a pressure in the injection body 30 to be smaller than a pressure in a first space 101, thus turning on a one-way valve by a pressure difference between the first space 101 and the injection body 30, and causing a drug stored in the first space 101 to be filled in the third space 301 of the injection body 30. The external force pulls the piston pull rod and compresses the constant force providing device 50 to enable the system to store a potential energy of the spring as a power source of a pump.

In the second operation state, the external force applied to the piston disappears, the film drug storage cavity and the syringe drug injection body are in an environment of an identical barometric pressure, and the pressure difference between both is completely provided by the constant force providing device. Under the circumstance, the compressed constant force spring pushes the piston to move to a side proximate to the second space 201, so that the syringe drug injection body becomes a pressure cavity, the one-way valve is turned off under a pressure in a forward direction, and the pressurized liquid drug cannot flow through the one-way valve, but only flow to a flow rate limiter and a quantifying syringe.

Fig. 4 is an enlarged schematic diagram of a one-way valve according to an embodiment of the disclosure. As shown in the figure, a unidirectional switch 20 has a second space 201, and a sealing gasket 211 (e.g., a silica gel sealing gasket), a steel ball 212 and a spring 213 are arranged along an extension direction of the second space 201. The sealing gasket 211 is provided with an opening allowing a liquid drug to circulate between a drug accommodation space of a drug storer 10 and a space of a unidirectional switch. The steel ball 212, pushed by the spring 213, presses and seals the opening. The second space 201 has a passageway 214 wound by a passageway wall 215, so that the second space 201 is in fluid communication with a third space 301 of the injection body 30 through the passageway 214. As shown in the figure, the spring 213 is arranged against an end of the passageway wall 215.

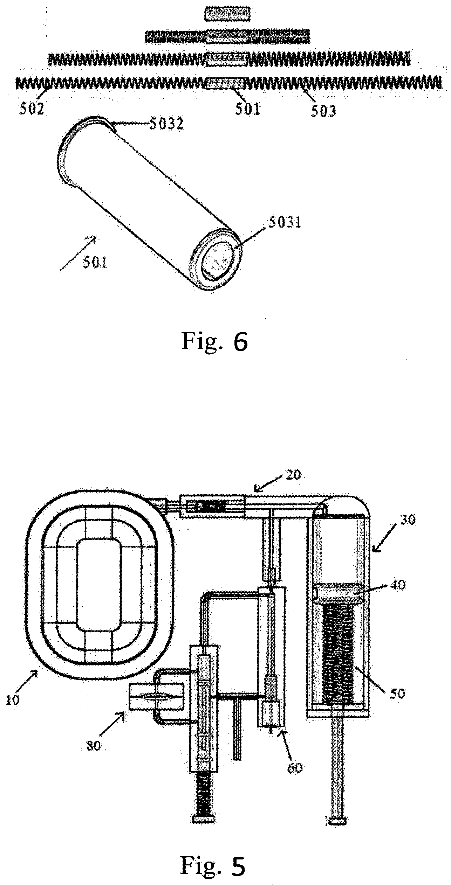

Fig. 5 is a schematic diagram showing an injection device having a flow rate limiter 60 and a quantifying syringe 80 according to an embodiment of the disclosure. A constant force providing device 50 is directly fixed to a drug piston 40. The constant force providing device 50 can apply a constant force to the drug piston 40, pushing the piston 40 to move along an extension direction of a space (a third space 301), and pushing out a drug in an injection body 30. An injection body 30 independently driven through pressurization by a constant force spring effects injection to a patient at a constant flow rate through the flow rate limiter 60. The injection body 30 driven through pressurization by the constant force spring effects timely quantitative injection to a patient by a manual quantifying syringe 80.

In an embodiment, a constant force providing device 50 may be, e.g., a constant force spring. As shown in Fig. 6, the constant force spring includes a sleeve 501, and a first spring 502 (small) and a second spring 503 (large) respectively arranged inside the sleeve 501. The sleeve may be made of metal or plastic, such as ABS. A projection 5031 for limiting the second spring 503 and a projection 5032 for limiting the first spring 502 are provided at an end of the sleeve 501. The first spring 502 and the second spring 503 are connected adjacently inside the sleeve, and the first spring 502 (small) has a smaller cross-section than a cross-section of the second spring 503 (large), so that when the two springs are compressed, the compressed first spring 502 (small) is compressed within the compressed second spring 503 (large) . The two springs may be made of an ordinary spring steel or stainless steel, and have an identical stiffness coefficient. Fig. 6 further separately shows a schematic diagram of a configured structure of the sleeve 501 and a state of gradually compressing the first spring 502 (small) and the second spring 503 (large).

Because of size limitation, in certain cases, the space left to the spring is limited in the device. The constant force spring design needs to increase the free length of the spring as much as possible, so the compressed length of the spring is increased accordingly. The design of a spring sleeved inside another spring as shown in the figure is used: a spring having a larger diameter is in the outside, a spring having a slightly smaller diameter is in the inside, and the two springs are connected end to end with a connecting sleeve to greatly increase the length of the springs under the condition of a given size.

Assume that a free length of the spring having a large diameter is L1, and its compressed length is L0; a free length of the spring having a small diameter is L2, and its compressed length is also L0; and a length of the connecting sleeve is approximately equal to L0. Then a total free length of the assembled spring is L1+L2-L0, its compressed length is L0, and the total compressed length in the working state is L=L1+ L2-2×L0.

If L1=200MM, L2=150MM and L0=25MM, then L=200+150-50=300MM.

If the spring stroke is △L=20MM,
then the error=△L/L=20÷300=0.067=±3.3%.

If a single spring, instead of a composite spring design, is used, then
L=200-25=175 mm.

When the same stroke is ΔL=20MM,

a much larger error=ΔL/L=20÷175=0.114=±5.7% will be obtained.

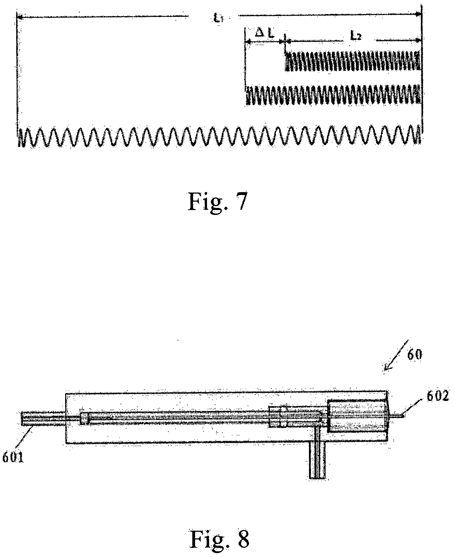

FIG. 7 shows a schematic diagram of a spring useful for a constant force providing device according to an embodiment of the disclosure. The spring R has a small stiffness coefficient and a high compression ratio to facilitate achieving the purpose of an approximate constant force by a very short operating stroke of the spring R.

As shown in Fig. 7, the free length of the spring R is L1, the length of the spring is compressed to L2 when the spring R works under the action of a drug piston 40, and the working length of the spring is ΔL. When a spring having a stiffness coefficient of k is used, the pressure difference (error) in the actual working process may be calculated by the following formula:

$$\Delta F = F_{\max} - F_{\min} = k(L_1 - L_2) - k(L_1 - L_2 - \Delta L) = k\Delta L$$

As can be seen from the formula, the error may be reduced by reducing the stiffness coefficient and shortening the operating stroke ΔL. Therefore, in an embodiment of the invention, the spring having a small stiffness coefficient is used and a short stroke is used to effect an approximate constant force spring.

In an embodiment, a flow rate limiter 60 may have a capillary structure to precisely and constantly effect the flow rate by utilizing a flow rate limiting effect of the capillary, i.e., compensating for an inner diameter error using a length. When a fluid passes through a tubule having a round cross-section very slowly, such a flow is laminar flow. An exact solution of the laminar flow in the round tube may be obtained using an analytical method. The flow rate of the fluid in the round tube is associated with the pressure difference at both ends of the tube, the viscosity of the fluid per se, the length of the tube and the diameter of the round tube. When it is used as a flow rate limiter, the exact flow rate may be calculated by the following equation:

$$Q=0.014625\times(D4\times\Delta P)/(\eta\times L).$$

Q: Flow rate (μL/day)

ΔP: Pressure drop on the flow rate limiter 50 (psi)

η: Viscosity of a liquid passing through the flow rate limiter 50 (cp)

L: Length of the round tube of the flow rate limiter 50

D: Radius of the round tube ($\mu$m)

In another embodiment, as shown in Fig. 8, the flow rate limiter 60 may include a capillary 601 and a column (such as metal wire) 602 inserted into the capillary 601. A gap allowing a liquid drug to circulate is formed between the capillary 601 and the inserted column 602. For example, the capillary 601 may be, for instance, a round tube, the column 602 may be, for instance, a cylinder, and then the round tube and the inserted cylinder form a tubular flow rate limiter of an annular flow.

The flow rate limiter 60 adjusts the flow rate of the flow rate limiter through a stack length L of the round tube and the cylinder. It should be understood that the stack length L may be preset based on specific needs. When the flow rate is slow (laminar flow), the liquid flow rule in the annular flow tube is:

$$Q = (\pi \, \Delta P)/8\eta L[R_o^4 - R_i^4 - (R_o^2 - R_i^2)^2/Ln(R_o/R_i)]$$

Q: Flow rate

$\Delta p$: Pressure drop on the flow rate limiter

H: Viscosity of the liquid flowing through the flow rate limiter

L: Length of the flow rate limiter: stack length of the round tube and the cylinder

Ro: Radius of the round tube

Ri: Radius of the cylinder

Ln: logarithm of a natural number to base e

Further returning to Fig. 5, an indwelling needle hose 70 may also be provided in the fluid outflow direction of the flow rate limiter 60 for transporting a liquid drug to a human body by a needle connected to the interface. The needle hose 70 may be in fluid communication with the flow rate limiter 60 by conventional connection means. By a flow rate limiting effect of the flow rate limiter 60, the liquid drug accommodated in the injection body 30 may be continuously discharged at a constant flow rate under a pressure provided by the constant force spring via the indwelling needle hose 70.

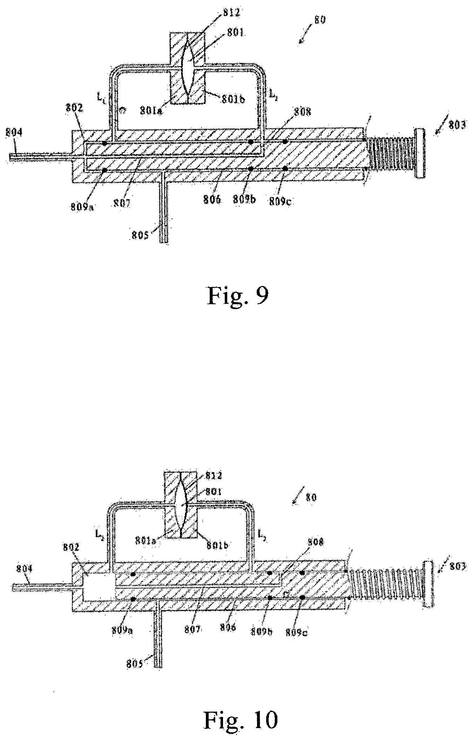

FIG. 9 and FIG. 10 show an enlarged view of the quantifying syringe 80 according to FIG. 5. The quantifying syringe 80 includes a volume body 801 having a first passage L1 and a second passage L2. The volume body 801 includes a first casing 810a having a concave, a second casing 810b having a concave, and a film 812 arranged between the first casing 810a and the second casing 810b. The shape of the first casing 810a corresponds to the shape of the second casing 810b. The film 812 is hot pressed between the first casing 810a and the second casing 810b by a hot pressing technology, causing the two casings to be pasted and isolate the volume body 801 into a first volume body and a second volume body. Thus, the volume body 801 has a one-way valve function. The quantifying syringe 80 further includes a fluid pipe having a fluid passage 802, a spring pull rod 803 having a cylindrical valve, a pressurized liquid inlet 804, a normal pressure liquid outlet 805 and a drug accommodation cavity 806. The pull rod 803 includes a center hole 807, an opening 808 connected to the center hole 807, and sealing rings 809a-c. The pressurized liquid inlet is in fluid communication with the center hole 807 of the pull rod 803. A gap 806 may be formed between a passageway 805 and the pull rod 803 by sealing using the sealing rings 809a and 809b.

As shown in FIG. 9, when the pull rod 803 is pushed by overcoming a spring force, the opening 808 on the pull rod 803 and connected to the center hole 807 is in fluid communication with the second passage L2 of the volume body 801. A pressurized liquid flows into the second volume body 801b through the opening 808 to push the film 812 to move towards the first volume body 801, thus discharging a liquid accommodated in the first volume body 801a to the passageway 805 through the first passage L1. The discharged liquid is discharged through the liquid outlet 805 via the cavity 806 between the fluid passage 802 and the pull rod 803.

As shown in Fig. 10, when the spring pull rod is released to return to an initial position, the opening 808 on the pull rod 803 is disconnected with the second passage L2 of the volume body 801, and the first passage L1 of the volume body 801 is in fluid communication with the pressurized liquid inlet, so that a fluid flows into the first volume body 801a through the first passage L1. A liquid flowing into the first volume body 801a pushes the film 812 to move towards the second volume body 801b, thus discharging a liquid accommodated in the second volume body 801b through the second passage L2. The discharged liquid is discharged through the liquid outlet 805 via the cavity 806 between the fluid passage 802 and the pull rod 803.

Therefore, the liquid in the volume body 801 is discharged into a corresponding pipe when the pressure difference is transferred each time. In a round of operation of pushing the pull rod 803 to compress a spring and then the spring automatically returning, two volume bodies of a liquid drug will be discharged through the normal pressure liquid outlet 805 via the pipe. An impetus driving the liquid drug is a pressure of the liquid at the inlet 804. Therefore, the inlet 804 of the device must be operated by a pressurized liquid. The number of times of pushing the pull rod 802 determines a volume of the discharged liquid. The device does not have a leak passageway, so all sealing elements may be very easily tested and verified during debugging. Because the liquid drug discharged each time is only associated with the volume of the cavity formed by injection molding, but is not associated with operation and environmental parameters (such as pressure, speed and temperature), the volume of the discharged liquid drug is very accurate.

When it is used, for example, two units of insulin may be injected by pushing the spring once and after the spring returns. For example, if pre-meal injection of eight units of insulin is required, then it is only necessary to push the pull rod 803 four times.

According to an embodiment described herein, a design of recharging both a liquid drug and a potential energy of a spring may be achieved by manually (or electrically) pulling a piston pull rod. Timely quantitative injection to a patient may be accurately realized using a safety design of mutually deadlocking a film-partitioning shallow arc-shaped cavity having a fixed volume and a communicating valve, as well as a design of fitting between a cylindrical communicating valve and a film-partitioning arc-shaped cavity. A fitting between a film storage cavity having a large volume and zero resistance and a cylindrical injection body having a small volume enables a constant force spring to be an easily realized design.

In order to further increase the volume of the injection device, a refillable spring-driven pump controlled by a control valve may be further provided.

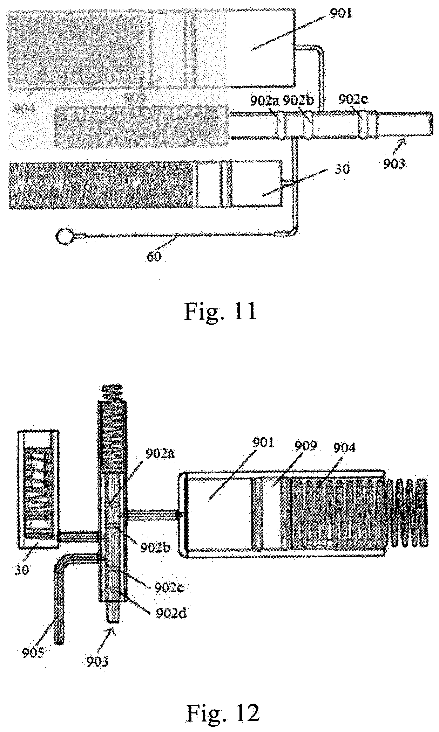

FIG. 11 shows a schematic diagram of a refillable spring-driven pump controlled by a control valve.

As shown in Fig. 11, a refillable spring-driven pump 90 controlled by a valve may include a drug accommodation cavity 901 in fluid communication with an injection body 30 through a control valve 903. A piston 904 is arranged in the drug accommodation cavity 901, and a non-constant force spring 909 for driving the piston 904 to move along the accommodation cavity 901 is arranged at an end of the piston distal to the control valve 903. A spring force of the non-constant force spring 909 needs to be enough to overcome a spring force of the drug injection body to inject a liquid drug into the injection body 30.

The control valve 903 includes a cavity 906 and a second piston 907 arranged in the cavity 906. A plurality of sealing rings 902a, 902b and 902c are arranged between the second piston 907 and the cavity 906. A spring device 908 is arranged at an end of the second piston 907. When it is necessary to inject a liquid drug into the drug injection body 30, the second piston 907 is pressed at a second end of the second piston 907 from an initial position shown in Fig. 10, causing the spring device 908 to be compressed. Under the circumstance, the drug accommodation cavity 901 is in fluid communication with the drug injection body 30 through a gap between the second sealing ring 902b and the third sealing ring 902c, causing the drug to flow from the drug accommodation cavity 901 into the injection body 30. When the piston is released, the compressed spring device 908 drives the second piston to return to its initial position, thus isolating the drug accommodation cavity 901 from the injection body 30 through the second sealing ring 902b.

In the spring-driven pump shown in Fig. 11, a valve return spring of the control valve doesn't need to be a constant force spring, and is OK as long as it can overcome a friction force of a sealing ring to enable the control valve to return to the initial position after being pressed.

Fig. 12 shows another embodiment of the spring-driven pump according to Fig. 11.

Different from the embodiment shown in Fig. 11, a plurality of sealing rings 902a, 902b, 902c and 902d are arranged between the second piston 907 and the cavity 906, and an indwelling needle catheter 905 in fluid communication with the injection body 30 is connected to the cylindrical control valve 903. When a liquid drug is injected into the drug injection body 30, the second piston 907 is pressed at an end of the second piston 907 opposite to the spring device from the initial position, causing the spring device 908 to be compressed (as shown in Fig. 11). Under the circumstance, the drug accommodation cavity 901 is in fluid communication with the drug injection body 30 through the gap between the second sealing ring 902b and the third sealing ring 902c, and the indwelling needle catheter 905 may be respectively isolated from the drug accommodation cavity 901 and the drug injection body 30 through the third sealing ring 902c, thereby ensuring the drug not to flow into the indwelling needle catheter 905 when the liquid drug is injected. When the second piston 907 is released, the compressed spring device 908 drives the second piston 907 to return to the initial position, thus isolating the drug accommodation cavity 901 from the drug injection body 30 through the second sealing ring 902b, and the injection body 30 is in fluid communication with the indwelling needle catheter 905 through the gap between the second sealing ring 902b and the third sealing ring 902c, enabling the liquid drug filled in the drug injection body to be injected into a patient's body through the indwelling needle.

With such a design of driving the drug injection body by the drug accommodation cavity, the foregoing flow rate limiter may be arranged at an outlet of the drug injection body to achieve the purpose of sustained release of a drug.

This may realize injection of an essential drug (such as insulin) at a desired constant flow rate. If a flow rate limiter is not arranged at an outlet of the drug injection body, then a given quantity may be injected instantly at a time when the control valve is operated. Such an injection may satisfy the need for, e.g., pre-meal drug injection.

For the flow rate limiter, because the error of the whole system is calculated based on the injection body volume, instead of the flow rate, the precision of the flow rate limiter, the precision of the injection body spring and the like are no longer important. Therefore, it is not necessary to design the flow rate limiter to be adjustable, nor is a debugging process required in the whole process of assembling the device. The accuracy of the system may be guaranteed by the volume of the drug injection body.

A drug injection device and a drug injection device system according to the embodiments of the disclosure are described above. However, it should be understood that the above description only provides embodiments to implement the invention, other than to limit the invention. Any modification, equivalent replacement, improvement and the like of the invention within the spirit and principle of the invention shall be included in the scope of protection of the invention.

**Claims**

1. An injection device comprising:

   a drug storer (10) having a first space (101) for storing a drug;
   a unidirectional switch (20) having a second space (201) in fluid communication with the first space (101); and
   an injection body (30) having a third space (301) in fluid communication with the second space (201), wherein the third space (301) is provided with a first piston (40) adapted to move along the third space (301), and to pump the drug stored in the first space (101) into the third space (301),
   wherein the drug storer (10) comprises a base (102) and a head cover (103), and at least one of the base (102) and the head cover (103) is formed by a flexible film, thereby forming the first space (101).

2. The injection device according to claim 1, wherein the base (102) has a concave, and the head cover (103) is formed by the flexible film, thereby enabling the film and the concave to form the first space.

3. The injection device according to claim 1, wherein both the base (102) and the head cover (103) are formed by the flexible film.

4. The injection device according to claim 2, wherein the flexible film is pasted to the base by a hot pressing technology.

5. The injection device according to claim 3, wherein the flexible films are pasted by a hot pressing technology.

6. The injection device according to claim 1, wherein a one-way valve is arranged in the second space (201), and wherein the one-way valve is arranged on a side proximate to the storage cavity (10) in the second space (201).

7. The injection device according to claim 1, wherein the unidirectional switch (20) comprises a sealing gasket (211), a stopper (212) and a spring (213) successively arranged along an extension direction of the second space (201),

   wherein the sealing gasket (211) has an opening, and
   wherein the stopper (212) is arranged against the spring (213), and after the drug stored in the first space (101) is pumped into the third space (301), the stopper (212) seals the opening of the sealing gasket under the spring force of the spring (213).

8. The injection device according to claim 6, wherein on a side of the second space (201) distal to the storage cavity (10), a constant force providing device (50) connected to the piston (40) is arranged within the third space (301) to push the piston to discharge the drug pumped into the third space (301).

9. The injection device according to claim 7, wherein the constant force providing device (50) comprises a constant force spring, and the second space (201) has a first operation state and a second operation state,
   in the first operation state, a pressure in the second space (201) is decreased by compressing the constant force providing device (50), causing a pressure difference between the first space and the second space to push the spring (213) to turn on the unidirectional switch (20), and pumping the drug stored in the first space (101) into the third space (301) of the injection body (30), and
   in the second operation state, the compressed constant force providing device (50) provides a constant urging force to the first piston (40), causing the first piston (40) to move towards a direction of the second space (201), so that the pressure in the second space (201) is increased to compress the spring (213), thus turning off the unidirectional switch (20).

10. The injection device according to claim 1, wherein the base (102) forms a hard plastic base by polypropylene.

11. The injection device according to claim 9, wherein the constant force spring comprises:

   a sleeve (501), and
   a first spring (502) and a second spring (503) arranged inside the sleeve (501),
   wherein the first spring (502) has a smaller cross-section than a cross-section of the second spring (503), so that when the first spring (502) and the second spring (503) are compressed, the compressed first spring (502) is compressed within the compressed second spring (503).

12. The injection device according to claim 9, wherein the constant force spring has a small stiffness coefficient and a high compression ratio.

13. The injection device according to any one of claims 1 to 12 further comprising a flow rate limiter (60) arranged in a drug fluid receiving direction of the injection body (30) for limiting a flow rate of a liquid drug pushed out from the injection body (30).

14. The injection device according to claim 13, wherein the flow rate limiter (60) has a capillary structure to precisely and constantly effect the flow rate of the liquid drug by utilizing a flow rate limiting effect of the capillary.

15. The injection device according to claim 13, wherein the flow rate limiter comprises:

   a tubule, for receiving the liquid drug pushed out from the injection body (30); and
   a column, arranged in the tubule to form a gap with the inner wall of the tubule allowing the received liquid drug to pass through, wherein
   the length of the column within the tubule is preset, thus adjusting the flow rate of the liquid drug passing through the gap.

16. The injection device according to claims 1 to 12 further comprising:

   a quantifying syringe (80), arranged in a drug fluid receiving direction of the injection body (30) for accommodating a needed quantity of a liquid drug pushed out from the injection body (30).

17. The injection device according to claim 16, wherein the quantifying syringe (80) comprises:

   a volume body (801), provided with a first passage and a second passage on both sides thereof;
   a fluid pipe, having a fluid passage (802) in fluid communication with the volume body (801) through the first passage and the second passage;
   a pull rod (803), provided with a passageway (807), an opening (808) in fluid communication with the passageway (807), and sealing rings (809a, 809b, 809c);
   a pressurized liquid inlet (804), in fluid communication with the fluid passage (802) and the passageway (807) ; and
   a normal pressure liquid outlet (805);
   wherein the pull rod (803) moves along a longitudinal direction of the fluid passage (802), a gap is formed between the pull rod (803) and the fluid passage (802), and a drug accommodation cavity (806) is formed by the sealing rings (809a, 809b) between the pull rod (803) and the fluid pipe, and
   the normal pressure liquid outlet (805) is in fluid communication with the cavity (806), so that a liquid discharged from the volume body (801) to the cavity (806) via the first passage or the second passage is discharged through the normal pressure liquid outlet (805).

18. The injection device according to claim 17, wherein the volume body (801) comprises a first casing (810a) having a concave, a second casing (810b) having a concave, and a film (812) arranged between the first casing (810a) and the second casing (810b).

19. The injection device according to claim 18, wherein when the pull rod (803) is pushed, a pressurized liquid flows into the volume body (801) through the opening (808) via the second passage, to push the film (812) to move against the first casing (810a), thus discharging a liquid accommodated in the volume body (801) through the first passage (L1), and discharging the liquid discharged from the volume body (801) through the liquid outlet (805) via the cavity (806), and

wherein when the pull rod (803) is pulled and then released, a pressurized liquid flows into the volume body (801) through the fluid passage (802), to push the film (812) to move against the second casing (810b), thus discharging a liquid accommodated in the volume body (801) through the second passage (L2), and discharging the liquid discharged from the volume body (801) through the liquid outlet (805) via the cavity (806).

20. The injection device according to claims 1 to 12 further comprising:

a drug accommodation cavity (901), in fluid communication with the injection body (30) through a control valve (903),

wherein a liquid drug in the drug accommodation cavity (901) is controlled by the control valve (903) and controllably filled in the injection body (30).

21. The injection device according to claim 20, wherein the control valve (903)comprises:

a cavity (906),

a second piston (907) arranged in the cavity (906), and

a second sealing ring (902b) and a third sealing ring (902c) arranged between the second piston (907) and the cavity (906),

wherein a spring device (908)is arranged at an end of the second piston (907), when the second piston (907) is pressed at a second end of the second piston (907), the spring device (908) is compressed, and the drug accommodation cavity (901) is in fluid communication with the injection body (30) through a gap between the second sealing ring (902b) and the third sealing ring (902c) ; and

when the piston is released, the compressed spring device (908) drives the second piston to return to an initial position, thus isolating the drug accommodation cavity (901) from the injection body (30) through the second sealing ring (902b).

22. The injection device according to claim 21 further comprising:

an indwelling needle catheter (905), in fluid communication with the injection body (30) through the control valve (903),

when the second piston (907) is pressed at a second end of the second piston (907), the spring device (908) is compressed, the drug accommodation cavity (901) is in fluid communication with the injection body (30) through the gap between the second sealing ring (902b) and the third sealing ring (902c), and the indwelling needle catheter (905) is respectively isolated from the drug accommodation cavity (901) and the injection body (30) through the third sealing ring (902c); and

when the second piston (907) is released, the compressed spring device (908) drives the second piston (907) to return to the initial position, thus isolating the drug accommodation cavity (901) from the injection body (30) through the second sealing ring (902b), and the indwelling needle catheter (905) is in fluid communication with the injection body (30) through the gap between the second sealing ring (902b) and the third sealing ring (902c).

23. The injection device according to claim 1, wherein the drug in the drug storer (10) is distributed through the unidirectional switch (20) to the injection body (30) to realize quantitative output of the drug.

24. A quantifying syringe (80) comprising:

a volume body (801), provided with a first passage and a second passage on both sides thereof;

a fluid pipe, having a fluid passage (802) in fluid communication with the volume body (801) through the first passage and the second passage;

a pull rod (803), provided with a passageway (807), an opening (808) in fluid communication with the passageway (807), and sealing rings(809a, 809b, 809c).

a pressurized liquid inlet (804), in fluid communication with the fluid passage (802) and the passageway (807) ; and

a normal pressure liquid outlet (805);

wherein the pull rod (803) moves along a longitudinal direction of the fluid passage (802), a gap is formed between the pull rod (803) and the fluid passage (802), and a cavity (806) for accommodating drug is formed by the sealing rings(809a, 809b) between the pull rod (803) and the fluid pipe, and

the normal pressure liquid outlet (805) is in fluid communication with the cavity (806), so that a liquid discharged from the volume body (801) to the cavity (806) through the first passage or the second passage is discharged through the normal pressure liquid outlet (805) .

25. The quantifying syringe according to claim 24, wherein the volume body (801) comprises a first casing (810a) having a concave, a second casing (810b) having a concave, and a film (812) arranged between the first casing (810a) and the second casing (810b).

26. The quantifying syringe according to claim 25, wherein when the pull rod (803) is pushed, a pressurized liquid flows into the volume body (801) through the opening (808) via the second passage to push the film (812) to move against the first casing (810a), thus discharging a liquid accommodated in the volume body (801) through the first passage (L1), and discharging the liquid discharged from the volume body (801) through the liquid outlet (805) via the cavity (806),

wherein when the pull rod (803) is pulled and then released, a pressurized liquid flows into the volume body (801) through the fluid passage (802) to push the film (812) to move against the second casing (810b), thus discharging a liquid accommodated in the volume body (801) through the second passage (L2), and discharging the liquid discharged from the volume body (801) through the liquid outlet (805) via the cavity (806).

Fig. 1

Fig. 2

103

102

Fig. 3

211 212 213 201

20

Fig. 4

502      501      503

5032

5031

501

## Fig. 6

20

30

40

10

50

80

60

## Fig. 5

Fig. 7

Fig. 8

EP 3 332 819 A1


Fig. 9

Fig. 10

Fig. 11

Fig. 12

# INTERNATIONAL SEARCH REPORT

| International application No. |
|---|
| PCT/CN2016/092122 |

## A. CLASSIFICATION OF SUBJECT MATTER

A61M 5/145 (2006. 01) i

According to International Patent Classification (IPC) or to both national classification and IPC

## B. FIELDS SEARCHED

Minimum documentation searched (classification system followed by classification symbols)

A61M 5/-; A61M 39/-

Documentation searched other than minimum documentation to the extent that such documents are included in the fields searched

Electronic data base consulted during the international search (name of data base and, where practicable, search terms used)

CNPAT, CNKI, WPI, EPODOC: HE, Xiaojun, Inject+, Transfusion, Infusion, IV, Film, Membrane?, Septum, Diaphragm+, Alternat+,

Bag, Spring, Constant+

## C. DOCUMENTS CONSIDERED TO BE RELEVANT

| Category* | Citation of document, with indication, where appropriate, of the relevant passages | Relevant to claim No. |
|---|---|---|
| X | CN 101124004 A (MCKINLEY MEDICAL LLLP) 13 February 2008 (13.02.2008) description, page 6, the last paragraph to page 7, the last paragraph, page 8, paragraph 4, page 12, paragraph 4, page 14, the last paragraph, page 15, paragraph 5, page 19, paragraph 3, and figures la-lc | 1-6, 8, 10, 13, 14, 16, 23 |
| Y | CN 101124004 A (MCKINLEY MEDICAL LLLP) 13 February 2008 (13.02.2008) description, page 6, the last paragraph to page 7, the last paragraph, page 8, paragraph 4, page 12, paragraph 4, page 14, the last paragraph, page 15, paragraph 5, page 19, paragraph 3, and figures la-lc | 7, 9, 11, 12, 15, 20-22 |
| Y | US 2014309582 A1 (CHIN, SAE HOON et al.) 16 October 2014 (16.10.2014) description, paragraphs [0046]-[0048], and figures1, and 7 | 7, 9, 11,12 |

☒ Further documents are listed in the continuation of Box C.        ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 September 2016 | 26 October 2016 |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer KONG, Xiangyun Telephone No. (86-10) 52871085 |
|---|---|

Form PCT/ISA /210 (second sheet) (July 2009)

## INTERNATIONAL SEARCH REPORT

| | International application No. |
|---|---|
| | PCT/CN2016/092122 |

C (Continuation).       DOCUMENTS CONSIDERED TO BE RELEVANT

| Category* | Citation of document, with indication, where appropriate, of the relevant passages | Relevant to claim No. |
|---|---|---|
| Y | US 6569128 B1 (ADVANCED INFUSION CORPORATION) 27 May 2003 (27.05.2003) description, col. 4, lines 1-20, and figure 2b | 15 |
| Y | CN 101360524 A (SMITHS MEDICAL ASD INC.) 04 February 2009 (04.02.2009) description, page 3, the last paragraph to page 8, paragraph 2, and figures1-5 | 20-22 |
| X | US 5176642 A (MECTRA LABS, INC.) 05 January 1993 (05.01.1993) description, col. 4,line 19 to col. 8, line 46, and figures1-3, and 5 | 1-6, 10, 16, 23 |
| Y | US 5176642 A (MECTRA LABS, INC.) 05 January 1993 (05.01.1993) description, col. 4,line 19 to col. 8, line 46, and figures1-3, and 5 | 7, 20-22 |
| X | US 2015080852 A1 (STC. UNM) 19 March 2015 (19.03.2015) description, paragraphs [0016]-[0025], and figures 1 and 2 | 1-6, 10, 16, 23 |
| Y | US 2015080852 A1 (STC. UNM) 19 March 2015 (19.03.2015) description, paragraphs [0016]-[0025], and figures 1 and 2 | 7, 20-22 |
| A | WO 2010051079 A2 (CALIBRA MEDICAL, INC.) 06 May 2010 (06.05.2010) description, paragraphs [0047], [0051], [0066]-[0073], and figures 1-3, 7 and 8 | 17-19, 24-26 |
| A | US 2013204130 A1 (MERIT MEDICAL SYSTEMS, INC.) 08 August 2013 (08.08.2013) the whole document | 1-26 |
| A | US 4995864( IMED CORPORATION) 26 February 1991 (26.02.1991) the whole document | 1-26 |

Form PCT/ISA /210 (continuation of second sheet) (July 2009)

## INTERNATIONAL SEARCH REPORT
### Information on patent family members

| International application No. |
| --- |
| PCT/CN2016/092122 |

| Patent Documents referred in the Report | Publication Date | Patent Family | Publication Date |
| --- | --- | --- | --- |
| CN 101124004 A | 13 February 2008 | JP 4769253 B2 | 07 September 2011 |
| | | US 8372045 B2 | 12 February 2013 |
| | | AU 2005306461 B2 | 20 January 2011 |
| | | CA 2587525 A1 | 26 May 2006 |
| | | IL 183145 A | 30 November 2010 |
| | | AU 2005306461 A1 | 26 May 2006 |
| | | JP 2008520373 A | 19 June 2008 |
| | | IL 183145 D0 | 20 September 2007 |
| | | CN 101124004 B | 18 April 2012 |
| | | KR 101013538 B1 | 14 February 2011 |
| | | EP 1812096 A2 | 01 August 2007 |
| | | WO 2006055834 A2 | 26 May 2006 |
| | | US 2006122562 A1 | 08 June 2006 |
| | | KR 20070089176 A | 30 August 2007 |
| | | CA 2587525 C | 21 February 2012 |
| US 2014309582 A1 | 16 October 2014 | US 9162028 B2 | 20 October 2015 |
| | | US 2012191036 A1 | 26 July 2012 |
| US 6569128 B1 | 27 May 2003 | US 7022107 B1 | 04 April 2006 |
| | | US 2002115966 A1 | 22 August 2002 |
| | | US 6645183 B2 | 11 November 2003 |
| CN 101360524 A | 04 February 2009 | JP 4842330 B2 | 21 December 2011 |
| | | WO 2007084214 A1 | 26 July 2007 |
| | | US 2007221275 A1 | 27 September 2007 |
| | | JP 2009523535 A | 25 June 2009 |
| | | EP 1973589 A1 | 01 October 2008 |
| | | US 7637279 B2 | 29 December 2009 |
| | | CA 2635640 C | 21 January 2014 |
| | | CA 2635640 A1 | 26 July 2007 |

Form PCT/ISA /210 (patent family annex) (July 2009)

EP 3 332 819 A1

## INTERNATIONAL SEARCH REPORT
### Information on patent family members

| | International application No. |
|---|---|
| | PCT/CN2016/092122 |

| Patent Documents referred in the Report | Publication Date | Patent Family | Publication Date |
|---|---|---|---|
| US 5176642 A | 05 January 1993 | WO 9215350 A1 | 17 September 1992 |
| | | AU 1765392 A | 06 October 1992 |
| US 2015080852 A1 | 19 March 2015 | US 8920382 B1 | 30 December 2014 |
| | | US 2015141957 A1 | 21 May 2015 |
| WO 2010051079 A2 | 06 May 2010 | WO 2009158651 A2 | 30 December 2009 |
| | | US 8128597 B2 | 06 March 2012 |
| | | US 8361030 B2 | 29 January 2013 |
| | | US 7927306 B2 | 19 April 2011 |
| | | US 2009326456 A1 | 31 December 2009 |
| | | US 2009326455 A1 | 31 December 2009 |
| | | WO 2009158654 A2 | 30 December 2009 |
| | | us 2009326454 A1 | 31 December 2009 |
| | | EP 2349407 A2 | 03 August 2011 |
| | | EP 2349400 A2 | 03 August 2011 |
| US 2013204130 A1 | 08 August 2013 | US 9265877 B2 | 23 February 2016 |
| US 4995864 | 26 February 1991 | CA 2003402 A1 | 15 February 1991 |
| | | EP 0413069 A1 | 20 February 1991 |
| | | JP H0388978 A | 15 April 1991 |
| | | AU 621519 B2 | 12 March 1992 |
| | | AU 4671989 A | 28 February 1991 |

Form PCT/ISA/210 (patent family annex) (July 2009)